## Europäisches Patentamt

## European Patent Office

## Office européen des brevets

(19)

(11) Veröffentlichungsnummer: **0 166 979**
**B1**

(12) # EUROPÄISCHE PATENTSCHRIFT

(45) Veröffentlichungstag der Patentschrift:
24.02.88

(21) Anmeldenummer: **85106794.2**

(22) Anmeldetag: **03.06.85**

(51) Int. Cl.⁴: **A 61 F 2/02**, A 61 B 17/42

(54) **Kammer zur Aufnahme eines von einem menschlichen Eierstock abgeschiedenen Ei's und zur Weiterleitung desselben in die Gebärmutter.**

(30) Priorität: 05.06.84 AT 1847/84

(43) Veröffentlichungstag der Anmeldung:
08.01.86 Patentblatt 86/2

(45) Bekanntmachung des Hinweises auf die Patenterteilung:
24.02.88 Patentblatt 88/8

(84) Benannte Vertragsstaaten:
BE CH DE FR GB IT LI LU NL SE

(56) Entgegenhaltungen:
FR-A-2 110 697
FR-A-2 235 707
US-A-4 193 392
US-A-4 228 550

(73) Patentinhaber: **Zech, Herbert, Dr., Belruptstrasse 32, A-6900 Bregenz (AT)**
Patentinhaber: **Becker, Horst, Belruptstrasse 32, A-6900 Bregenz (AT)**

(72) Erfinder: **Zech, Herbert, Dr., Belruptstrasse 32, A-6900 Bregenz (AT)**

(74) Vertreter: **Hefel, Herbert, Dipl.- Ing., Egelseestrasse 65a Postfach 61, A-6800 Feldkirch (AT)**

EP 0 166 979 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung bezieht sich auf eine Kammer zur Aufnahme eines von einem menschlichen Eierstock abgeschiedenen Ei's und zur Weiterleitung desselben in die Gebärmutter.

Frauen, die von Geburt anormale Eileiter besitzen, oder solche Frauen, bei denen aufgrund einer Erkrankung oder einer Eileiterschwangerschaft die Eileiter operativ entfernt werden mußten, können auf natürlichem Wege keine Kinder empfangen, obgleich ihre Eierstöcke in naturgegebener Weise arbeiten, d. h. Eier werden gebildet, reifen heran und werden in der Folge beim sogenannten Eisprung freigegeben. Die reifen, gesprungenen Eier können jedoch wegen oben genannter Ursachen nicht auf natürliche Weise befruchtet werden und in die Gebärmutter gelangen. Diese Eier gelangen vielmehr in die freie Bauchhöhle und werden hier vom Körper absorbiert.

Um den Wunsch nach einem eigenen Kinde zu erfüllen, hat die medizinische Wissenschaft die sogenannte künstliche Befruchtung - in Vitro-Fertilisation - entwickelt. Nach diesem Verfahren wird das herangereifte Ei dem Körper entnommen, außerhalb des Körpers befruchtet und dann wird das befruchtete Ei in die Gebärmutter transferiert, wo es sich naturgemäß einnistet, weiterentwickelt und hier bis zur Geburt ausgetragen wird. Die Entwicklung dieser Methode steht trotz spektakulärer Berichte in den Medien noch in den Anfangsstadien (Erfolgsquoten 10 bis 20 % pro Versuch) - in den letzten 3 bis 4 Jahren sind jedoch sicherlich deutliche Fortschritte erzielt worden. Dennoch ist unbestritten, daß die hier praktizierte Methode von einem natürlichen Zeugungsakt doch sehr weit entfernt liegt, abgesehen von all den ethischen Fragen, die in diesem Zusammenhang auftauchen und noch nicht bewältigt sind. Hier setzt nun die Erfindung ein, die darauf abzielt, eine Voraussetzung im weiblichen Körper zu schaffen, dank der auch eine Frau mit den eingangs aufgezeigten Mängeln über einen natürlichen Zeugungsakt ein Kind empfangen kann. Gemäß der Erfindung ist dazu eine Kammer vorgesehen, die als dünnwandiger, ballonartiger Hohlkörper ausgebildet ist mit einer Aufnahmeöffnung für die Einführung und Aufnahme eines Eierstockes und der Rand der Aufnahmeöffnung mit der Gebärmutteraußenseite verbindbar ist und seitlich der Aufnehmeöffnung eine trichterartige Ausstülpung vorgesehen ist, welche in einen schlauchartigen Fortsatz übergeht, der in die Eileitermündung der Gebärmutter einführbar ist.

Die Erfindung und ihre besondere Anwendung wird anhand der Zeichnung eingehend erläutert. Es zeigen:

Fig. 1 eine Ansicht von vorne;
Fig. 2 von der Seite (Blickrichtung Pfeil A in Fig. 1);
Fig. 3 von hinten;
Fig. 4 von der Seite (Blickrichtung Pfeil B in Fig. 1);
Fig. 5 einen Vertikalschnitt gesehen in Richtung der Pfeile V - V in Fig. 1 und
Fig. 6 einen Vertikalschnitt gesehen in Blickrichtung der Pfeile VI - VI in Fig. 1;
Fig. 7 eine Ansicht gemäß Fig. 1 mit einer Darstellung der durchlässigen Wandzonen;
Fig. 8 eine Ansicht direkt gegen die Aufnahmeöffnung (Blickrichtung Pfeil C in Fig. 2);
Fig. 9 einen Querschnitt im Bereich des nach innen vorspringenden Randes;
die Fig. 10, 11 und 12 zeigen die Kammer bestimmungsgemäß eingesetzt in Verbindung mit einer Gebärmutter und zwar von vorne, (d. h. bei liegender Patientin von den Beinen Richtung Kopf gesehen) von der Seite und von hinten;
Fig. 13 entspricht der Darstellung nach Fig. 11 jedoch bei geänderter Lage der Gebärmutter.

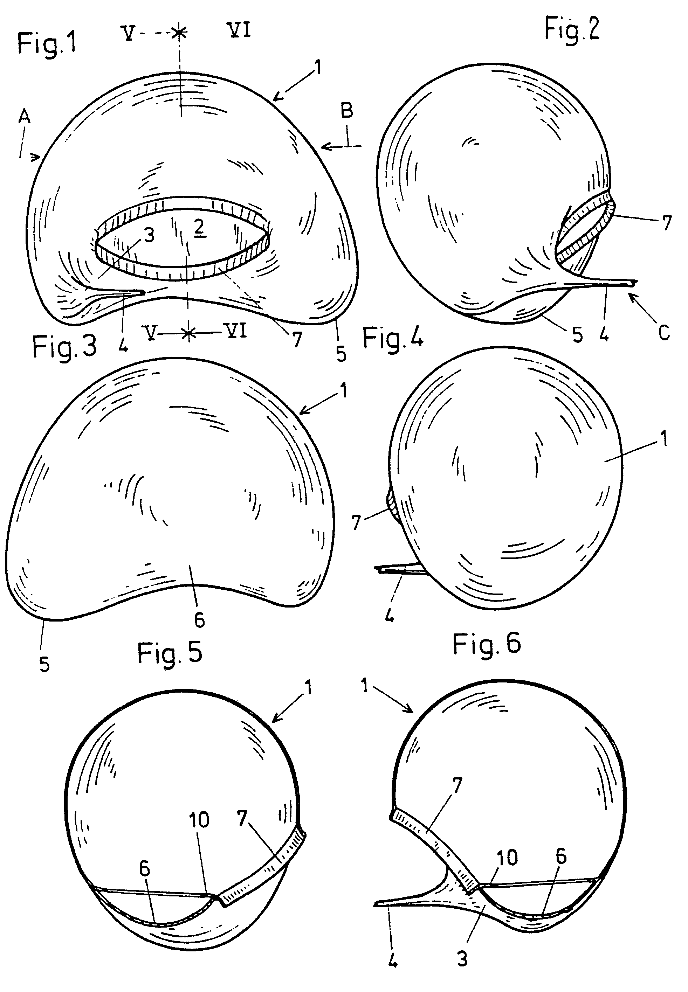

Vorerst wird anhand der Fig. 1 bis 9 die Ausgestaltung der Kammer im Detail erläutert. Diese Kammer ist als ballonartiger Hohlkörper 1 ausgebildet mit einer dünnen, aus einem körper- und gewebeverträglichen Kunststoff gefertigten Wandung, wobei zumindest die Innenfläche dieser Wandung sehr glatt ist. Das Wort "ballonartig" ist im gegenständlichen Falle so zu verstehen, daß die Kammer eine dünne Wand aufweist und ein zum Verhältnis des Volumens des Wandmaterials großes Raumvolumen umschließt, wobei die den Hohlkörper bildenden Wandungen gewölbt sind. Dieser Hohlkörper umschließt ein Volumen von ca. 55 bis 75 ccm. Der ballonartige Hohlkörper 1 besitzt eine Aufnahmeöffnung 2, von etwa ellipsenartiger Form, deren Achslängen ca. 3 bis 4 cm betragen. Die hier und im folgenden angegebenen Maße aind als Richtgrößen zu verstehen. Da der Hohlkörper 1 kein eindeutig definierbares Achssystem besitzt, wird hier und im folgenden die erwähnte Aufnahmeöffnung 2 sozusagen als Bezugsgröße gewählt, auf welche Lageangaben bezogen werden. Es ist dabei aber festzuhalten, daß der Rand dieser Aufnahmeöffnung 2 nicht unbedingt in einer geometrischen Ebene liegen muß, der Rand dieser Aufnahmeöffnung 2 kann auch räumlich verlaufen. Diese Aufnahmeöffnung 2 ist so groß bemessen, daß ohne Behinderung ein Eierstock üblicher Größe in diese Öffnung einführbar ist. Darauf wird aber dann im folgenden noch im Detail Bezug genommen.

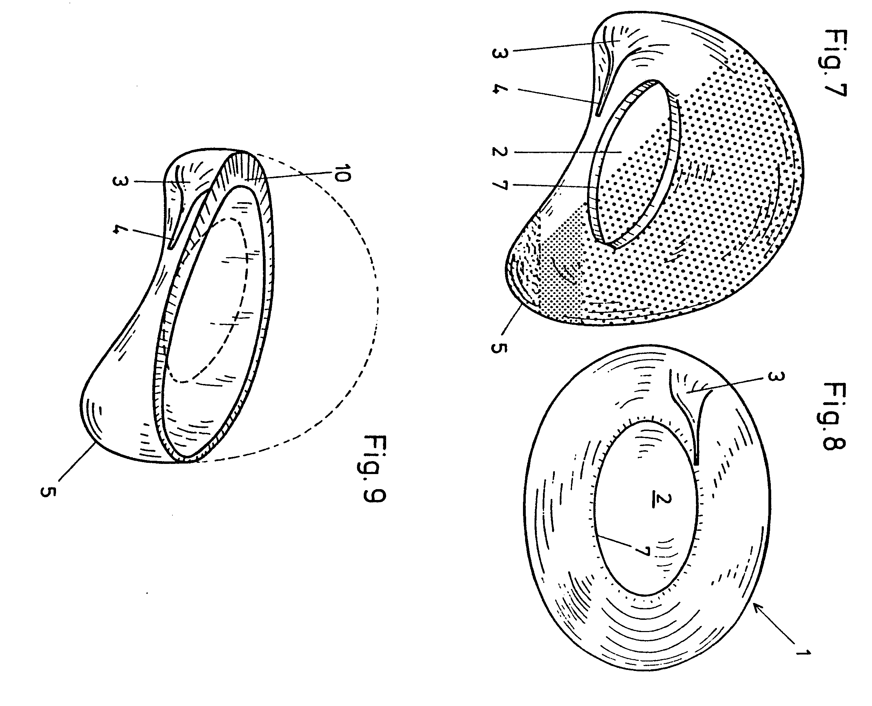

Wird die Kammer von vorne betrachtet (Fig. 1), wobei hier ihre erste Gebrauchslage gezeigt ist, bei welcher die Ebene der Aufnahmeöffnung 2 mit der Horizontalebene einen Winkel von ca. 40° einschließt (Fig. 2), so liegt seitlich der erwähnten Aufnahmeöffnung 2 und etwas hinter bzw. unter deren Längsmittelebene eine trichterartige Ausstülpung 3, welche in einen schlauchartigen Fortsatz 4 übergeht bzw. in einen solchen schlauchartigen Fortsatz 4 ausläuft. Auf der anderen Seite der Aufnahmeöffnung 2, in Fig. 1 auf der rechten Seite, und, bezogen auf deren Längsmittelebene, seitengleich mit der trichterartigen Ausstülpung 3 ist der Hohlkörper 1 zu einer flachen, muldenartig vertieften Zone 5 ausgestaltet. Die innere Wandung des Hohlkörpers, welche zwischen der trichterartigen Ausstülpung 3 und der vertieften Zone 5 und seitlich und unterhalb der Aufnahmeöffnung Z liegt, bildet einen die genannten Bereiche voneinander trennenden Wall 6 geringer Höhe. In der in Fig. 1 gezeigten Gebrauchslage der Einrichtung trennt dieser Wall die trichterartige Ausstülpung 3 von der muldenartigen Vertiefung. Diese muldenartige Vertiefung 5 hat bezogen auf die Oberkante dieses Walles 6 ein Fassungsvolumen von ca. 3 cm³. Diese muldenartige Vertiefung mit dem erwähnten Fassungsvermögen von 3 cm³ ist von einer glatten, undurchlässigen Wand begrenzt. Oberhalb dieser muldenartig flachen, vertieften, von einer undurchlässigen Wand begrenzten Zone ist die Wandung des Hohlkörpers durchlässig, vorzugsweise perforiert, wobei die Durchlässigkeit der Wandung des Hohlkörpers mit zunehmendem Abstand von dieser vertieften Zone 5 zunimmt. In Abflußrichtung und im Bereich der trichterartigen Ausstülpung ist die Wand jedoch nicht durchlässig. Zu diesem Zweck sind in der Wandung des Hohlkörpers zahlreiche Durchbrechungen oder Bohrungen vorhanden, die im Bereich der geringen Durchlässigkeit einen Bohrungsdurchmesser von ca. 0,5 mm aufweisen, im Bereich der erhöhten Durchlässigkeit jedoch einen Durchmesser bis zu 1,5 mm haben. Nur in der Fig. 7 sind diese beiden Bereiche ungleicher Durchlässigkeit durch Punkteraster bezeichnet. In die anderen Figuren sind diese Punkteraster nicht übernommen, um die Anschaulichkeit der Darstellung nicht zu beeinträchtigen.

Der Rand 7 der Aufnahmeöffnung 2 ist vorteilhafterweise aus einem anderen Material gefertigt als der Hohlkörper selbst, denn über diesen Rand wird der Hohlkörper mit der Außenseite der Gebärmutter fest verbunden. Die Fig. 1 bis 8 zeigen den Hohlkörper in zahlreichen verschiedenen Ansichten, um auf diese Weise dem Beschauer eine konkrete Vorstellung über sein Aussehen zu vermitteln. Das innere Volumen des Hohlkörpers ist so bemessen, daß ein Eierstock durchschnittlicher Größe, auch bei Vorhandensein sprungreifer Follikel, ohne Behinderung aufgenommen werden kann.

Aus der Schnittdarstellung nach Fig. 9 ist noch erkennbar, daß nach innen ein umlaufender Rand 10 vorspringt, der einerseits unterhalb der Aufnahmeöffnung 2 verläuft und andererseits oberhalb der trichterartigen Ausstülpung 3 und der muldenartigen Vertiefung 5.

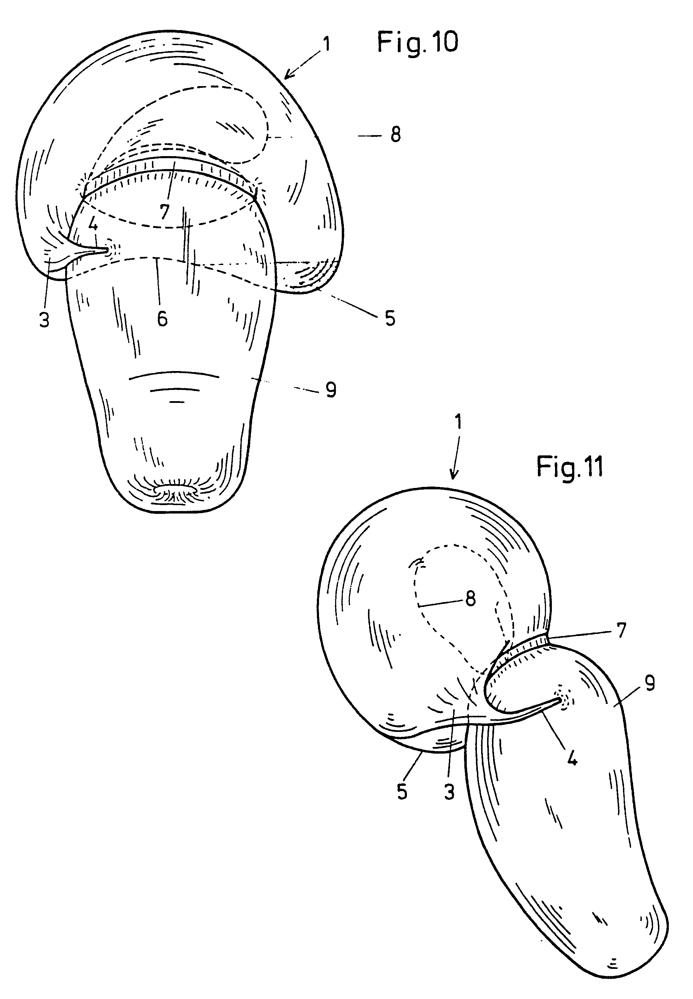

Im folgenden wird nun erörtert, wie diese Kammer bestimmungsgemäß angewandt wird und zwar im Zusammenhang mit den Fig. 10 bis 13, die die Anordnung der Kammer auf eine Gebärmutter zeigen. Es versteht sich, daß das, was im folgenden über den bestimmungsgemäßen Einsatz dieser Kammer ausgesagt werden wird, unter ärztlicher Kontrolle geschieht. Die vorstehend beschriebene Kammer wird operativ eingesetzt. Dazu wird als Vorbereitungsmaßnahme einer der beiden Eierstöcke 8 über die Gebärmutter 9 geklappt und daran fixiert, wie dies Fig. 10 von vorne veranschaulicht und nötigenfalls wird die Gebärmutter in einer sogenannten Aufrichtungsoperation fixiert. Nun wird die Kammer nach den Fig. 1 bis 9 eingebracht, wobei die Kammer so auf die Gebärmutter 9 aufgesetzt wird, daß der auf der Gebärmutter 9 fixierte Eierstock 8 zur Gänze in der Kammer liegt. Mittels des Randes 7 wird die Kammer nun mit der Gebärmutter fest verbunden, beispielsweise vernäht. Der schlauchartige Fortsatz 4 wird entsprechend abgelängt und das verbleibende Stück in die Öffnung der Gebärmutter 9 eingeführt, in welche ursprünglich der Eileiter gemündet hat. Dieser schlauchartige Fortsatz 4 ist so in seiner Länge zu bestimmen, daß er nicht in das Innere der Gebärmutter vorsteht.

Damit ist nun die Einrichtung für ihre funktionsgerechte Bestimmung vorbereitet. Die Fig. 11 zeigt die Lage der Gebärmutter der liegenden Frau. Es ist dabei erkennbar, und zwar aus den Fig. 10 bis 12, daß die seitliche muldenartig vertiefte Zone 5 tiefer liegt als die trichterartige Ausstülpung 3 und daß sich zwischen diesen beiden Bereichen der vorstehend beschriebene, trennende Wall 6 erstreckt.

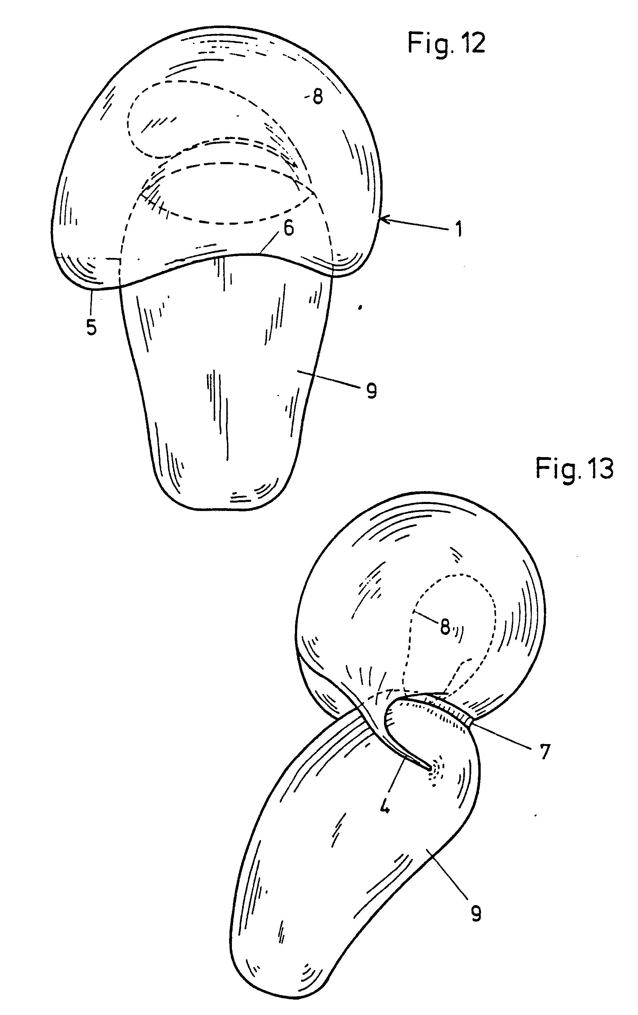

Im Eierstock 8 reift im monatlichen Zyklus mindestens ein Ei heran. Das reife Ei wird dann vom Eierstock freigegeben (Eisprung) und das nun freigegebene Ei gelangt zusammen mit der Follikelflüssigkeit in die Kammer 1 und zwar in die muldenartig vertiefte Zone 5. Pro Eisprung werden ca. 6 bis 8 ml Follikelflüssigkeit mit samt dem Ei frei. Bezogen auf das Eivolumen ist diese Flüssigkeitsmenge relativ groß und diese Flüssigkeitsmenge wäre imstande, das Ei wegzuspülen oder seine nachfolgende Einnistung in der Gebärmutter zu stören und zu beeinträchtigen. Die überschüssige Follikelflüssigkeit wird daher abgeleitet, sie kann durch die durchlässigen Wandbereiche der Kammer nach außen in die Bauchhöhle abfließen. Es verbleibt daher nur so viel Follikelflüssigkeit in der Kammer, wie diese in ihrer vertieften muldenartigen Zone aufzunehmen vermag (siehe Fig. 10). Hat die Frau nun Geschlechtsverkehr in Rückenlage, wobei die Gebärmutter die aus den Fig. 10 bis 12 ersichtliche Lage beibehält, so nimmt die Gebärmutter männlichen Samen auf. Diese können bei genügender Eigenbeweglichkeit und vorhandenem schleimigem Überzug der Innenflächen dieser Kammer bis zum Ei in der Mulde 5 gelangen und dieses befruchten. Erhebt sich anschließend die Frau, so geht die Gebärmutter aus der in Fig. 11 ersichtlichen Lage in jene über, die die Fig. 13 zeigt, d. h. die Kammer wird angehoben, wodurch die muldenartige Vertiefung 5 eine höhere Lage gegenüber der trichterartigen Ausstülpung 3 einnimmt, so daß die Follikelflüssigkeit, in der das Ei sich befindet, mit dieser nunmehr in die trichterartige Ausstülpung und in der Folge in den schlauchartigen Fortsatz 4 rinnt, wobei das Ei mitgespült wird. Das Ei gelangt nun so in die Gebärmutter, wo es, falls von dem Samen aus verschiedenen Gründen noch nicht befruchtet, noch nachträglich befruchtet werden kann. Unter Umständen können die Samen durch die trichterartige Ausstülpung nicht oder nicht in ausreichender Konzentration aufsteigen und zum Ei in der vertieften Mulde 5 gelangen. In diesem Falle wird das unbefruchtete Ei nach dem Aufstehen (ein Tag nach dem Eisprung) in die Gebärmutter gelangen und es kann auch hier noch von dem hier befindlichen Samen befruchtet werden. Das so befruchtete Ei nistet sich in der Gebärmutter ein und entwickelt sich in der Folge bis zur Geburt, welche auf natürliche Weise erfolgen kann, weiter. Wäre in der Kammer die vertiefte Zone 5 nicht vorgesehen, so würde das Ei gleich nach dem Eisprung in die Gebärmutter gelangen, aufgrund der zu erwartenden großen Menge an Follikelflüssigkeit jedoch auch im Falle der ordnungsgemäßen Befruchtung am Einnisten in der Gebärmutter eventuell gestört werden. Es wird daher als zweckmäßig erachtet, die eventuell überschüssige Follikelflüssigkeit rechtzeitig in die freie Bauchhöhe abzuleiten, was durch die vorgesehenen Maßnahmen gelingt.

Es können unter Umständen auch mehrere Eier gleichzeitig heranreifen und frei werden, insbesondere bei einer vorausgegangenen medikamentösen Behandlung, die bei Sterilitätspatienten oft zusätzlich notwendig ist wegen der Unterfunktion des Eierstockes. So ist es denkbar, daß bis zu 20 ml Follikelflüssigkeit anfallen wenn gleichzeitig drei Eier heranreifen. Diese relativ große Menge muß fürsorglich und rechtzeitig abgeleitet werden.

Die Durchbrechungen in der Wand der Kammer haben also die Aufgabe einerseits die überschüssige Follikelflüssigkeit abzuleiten. Aber auch das Ei soll zumindest die großporigen Durchbrechungen passieren können, falls es nicht nach dem Aufstehen der Frau nach unten über die trichterartige Ausstülpung in die Gebärmutter abfließen kann. Die großporigen Durchbrechungen sind auch ferner für das Ableiten von Menstrualblut gedacht. Dies kann aus der Gebärmutter bei eventuell zu großem Innendurchmesser und zu kurzer Länge der trichterartigen Ausstülpung zurückfließen und sollte auch dann vom Körper weggeschafft werden können.

Die vorstehend erläuterte Kammer übernimmt die Aufgabe des natürlichen Eileiters, indem das vom Eierstock freigegebene Ei aufgenommen und in der Folge in die Gebärmutter verfrachtet wird, und zwar mit einem Spülvorgang, zu welchem die beim Eisprung freiwerdende Follikelflüssigkeit herangezogen wird. Durch die Perforation der Kammerwandung ist die Follikelflüssigkeitsmenge für diesen Spülvorgang bestimmbar. Dank der erfindungsgemäßen Einrichtung kann die Frau über einen normalen Geschlechtsverkehr ein Kind empfangen und bis zu dessen Geburt austragen. Die Kammer wird zweckmäßigerweise aus einem Kunststoff gefertigt, wie er heute in der Medizin vielfach eingesetzt wird, aus einem gewebe- und körperverträglichen Material. Die Wandstärke und das Material für die Kammer sind so aufeinander abzustimmen, daß die Kammer eine gewisse Eigensteifigkeit besitzt, die Kammer also beim bestimmungsgemäßen Einsatz ihre Form beibehält, also den von außen durch die Eingeweide auf sie einwirkenden Druck ohne Formänderung zu erdulden vermag.

Die hier beschriebene Kammer kann eventuell ein Jahr lang im Körper einer Frau belassen werden. Falls während dieser Zeit bei sonst unauffälligem Befinden keine Schwangerschaft eintritt, ist ein längeres Zuwarten nicht vorgesehen. Grundsätzlich könnte jedoch bei Beschwerdefreiheit diese Kammer auch über Jahre im Körper verbleiben.

**Patentansprüche**

1. Kammer zur Aufnahme eines von einem menschlichen Eierstock abgeschiedenen Ei's und zur Weiterleitung desselben in die Gebärmutter, dadurch gekennzeichnet, daß sie als dünnwandiger, ballonartiger Hohlkörper (1) ausgebildet ist mit einer Aufnahmeöffnung (2) für die Einführung und Aufnahme eines Eierstockes (8) und der Rand (7) der Aufnahmeöffnung (2) mit der Gebärmutteraußenseite verbindbar ist und seitlich der Aufnahmeöffnung (2) eine trichterartige Ausstülpung (3) vorgesehen ist, welche in einen schlauchartigen Fortsatz (4) übergeht, der in die Eileitermündung der Gebärmutter (9) einführbar ist.

2. Kammer nach Anspruch 1, dadurch gekennzeichnet, daß der schlauchartige Fortsatz (4) gegen jene Seite des Hohlkörpers (1) gerichtet ist, an der die Aufnahmeöffnung (2) liegt.

3. Kammer nach Anspruch 1 oder 2, dadurch gekennzeichnet, daß bezogen auf die Aufnahmeöffnung (2) in etwa diametral zur trichterartigen Ausstülpung (3) der Hohlkörper (1) eine muldenartige, flache, vertiefte Zone (5) aufweist.

4. Kammer nach Anspruch 2 oder 3, dadurch gekennzeichnet, daß bezogen auf eine gedachte Mittelebene durch den Hohlkörper (1) sowohl die trichterartige Ausstülpung (3) wie auch die muldenartig vertiefte Zone (5) auf derselben Seite dieser Mittelebene liegen.

5. Kammer nach Anspruch 3, dadurch gekennzeichnet, daß die Wandung des Hohlkörpers (1), die in Gebrauchslage der Kammer oberhalb der muldenartigen, flachen, vertieften Zone (5) liegt, durchlässig vorzugsweise perforiert ist. (Fig. 7).

6. Kammer nach Anspruch 5, dadurch gekennzeichnet, daß die Durchlässigkeit der Wandung des Hohlkörpers (1) mit zunehmendem Abstand von der vertieften Zone (5) zunimmt.

7. Kammer nach einem der Ansprüche 1 bis 6, dadurch gekennzeichnet, daß die innere Wandung des Hohlkörpers (1), welche zwischen der trichterartigen Ausstülpung (3) und der vertieften Zone (5) und seitlich der Aufnahmeöffnung (2) liegt, einen die genannten Bereiche trennenden Wall (6) geringer Höhe bildet.

8. Kammer nach Anspruch 1, dadurch gekennzeichnet, daß das Volumen des Hohlkörpers ca. 55 bis 75 cm³ beträgt.

9. Kammer nach Anspruch 3, dadurch gekennzeichnet, daß das Fassungsvolumen der vertieften muldenartigen Zone in der Gebrauchslage der Kammer ca. 3 cm³ beträgt.

10. Kammer nach den Ansprüchen 5 und 6, dadurch gekennzeichnet, daß der an die vertiefte muldenartige Zone (5) anschließende Bereich zahlreiche Durchbrechungen oder Bohrungen aufweist mit einem Durchmesser von ca. 0,5 mm.

11. Kammer nach Anspruch 5 oder 6, dadurch gekennzeichnet, daß der eine erhöhte Durchlässigkeit aufweisende Bereich der Wandung zahlreiche Bohrungen oder Durchbrechungen besitzt mit einem Durchmesser von ca. 1,5 mm.

12. Kammer nach einem der Ansprüche 1 bis 4, dadurch gekennzeichnet, daß ein unterhalb der Aufnahmeöffnung (2) sowie oberhalb der trichterartigen Ausstülpung (3) und der muldenartig vertieften Zone (5) ein an der Innenwandung umlaufender und gegen das Innere des Hohlkörpers (1) vorspringender Rand (10) vorgesehen ist (Fig. 9).

13. Kammer nach Anspruch 12, dadurch gekennzeichnet, daß der Rand (10) im Bereich oberhalb der trichterartigen Ausstülpung (3) weiter vorspringt als in seinem übrigen Verlauf.

14. Kammer nach Anspruch 1, dadurch gekennzeichnet, daß der Hohlkörper (1) eine Größe hat, die etwa einem durchschnittlichen Hühnerei entspricht.

15. Kammer nach Anspruch 1, dadurch gekennzeichnet, daß der mit der Gebärmutter (9) verbindbare Rand (7) der Aufnahmeöffnung (2) aus einem anderen Material besteht als der eigentliche Hohlkörper.

**Claims**

1. A chamber for receiving an egg separated from a human egg supply and for passing it into the womb, characterised in that it is designed as a thin-walled, balloon-type hollow body (1) with a receiving aperture (2) for introducing and receiving a supply of eggs (8) and the edge (7) of the receiving aperture (2) is connectable to the outside of the womb and at the side of the receiving aperture (2) a funnel-type protuberance (3) is provided, which passes into a tubular extension (4), which can be introduced into the mouth of the fallopian tube of the womb (9).

2. A chamber according to claim 1, characterised in that the tubular extension (4) is orientated towards the side of the hollow body (1) against which the receiving aperture (2) lies.

3. A chamber according to claim 1 or 2, characterised in that relative to the receiving aperture (2) approximately diametrically to the protuberance (3) the hollow body (1) has a trough like, flat, recessed region (5).

4. A chamber according to claim 2 or 3, characterised in that with respect to a hypothetical central plane through the hollow body (1) both the funnel-like protuberance (3) and the trough-like recessed region (5) lie on the same side of this central plane.

5. A chamber according to claim 3, characterised in that the wall of the hollow body (1), which lies above the trough-like, flat, recessed region (5) in the operating position of the chamber, is preferably perforated so as to be permeable. (Fig. 7).

6. A chamber according to claim 5, characterised in that the permeability of the wall of the hollow body (1) increases as the distance from the recessed region (5) increases.

7. A chamber according to any one of claims 1 to 6, characterised in that the inner wall of the hollow body (1), which wall lies between the funnel-like protuberance (3) and the recessed region (5) and at the side of the receiving aperture (2), forms a low wall (6) separating the said regions.

8. A chamber according to claim 1, characterised in that the volume of the hollow body is approximately 55 to 75 cm³.

9. A chamber according to claim 3, characterised in that the volumetric capacity of the recessed, trough-like, region in the operating position is approximately 3 cm³.

10. A chamber according to claims 5 and 6, characterised in that the region adjacent the recessed trough-like zone (5) has a plurality of perforations or bores with a diameter of approximately 0,5 mm.

11. A chamber according to claim 5 or 6, characterised in that the region having increased permeability has a plurality of perforations or bores with a diameter of approximately 1,5 mm.

12. A chamber according to any one of claims 1 to 4, characterised in that below the receiving aperture (2) and above both the funnel-like protuberance (3) and the region (5) recessed in the manner of a trough an edge (10) surrounding the inner wall and projecting against the inside of the hollow body (1) is provided (Fig. 9).

13. A chamber according to claim 12, characterised in that the edge (10) in the region above the funnel-like protuberance (3) projects further than along the rest of its form.

14. A chamber according to claim 1, characterised in that the size of the hollow body (1) corresponds to an average chicken's egg.

15. A chamber according to claim 1, characterised in that the edge (7) of the receiving aperture (2) connectable to the womb (9) is made of a different material from the actual hollow body.

## Revendications

1. Chambre destinée à la réception d'une ovule produite par un ovaire humain et à la conduction de celle-ci dans l'utérus, caractérisée en ce qu'elle consiste en un corps creux à parois minces (1) en forme de ballon comportant une ouverture d'admission (2) pour l'introduction et la réception d'un ovaire (8), le bord (7) de l'ouverture d'admission (2) étant reliable à l'extérieure de l'utérus et en ce qu'une excroissance en forme d'entonnoir (3) est prévue latéralement par rapport à l'ouverture d'admission (2), excroissance (3) qui se poursuit dans un prolongement tubulaire (4) qui est introductible dans l'embouchure de la trompe utérine (9) dans l'utérus (9).

2. Chambre selon la revendication 1 caractérisée en ce que le prolongement tubulaire (4) est orienté vers le côté du corps creux (1), qui comporte l'ouverture d'admission (2).

3. Chambre selon la revendication 1 ou 2 caractérisée en ce que le corps creux (1) comporte une zone (5) enfoncée, plate, en forme d'auge, approximativement diamétralement opposée à l'excroissance en forme d'entonnoir (3).

4. Chambre selon la revendication 2 ou 3 caractérisée en ce que l'excroissance en forme d'entonnoir (3) ainsi que la zone enfoncée en forme d'auge (5) se situent d'un même côté d'un plan médian virtuel traversant le corps creux (1).

5. Chambre selon la revendication 3 caractérisée en ce que la paroi du corps creux (1) qui, en position d'utilisation de ladite chambre, se trouve au-dessus de la zone enfoncée plate en forme d'auge (5), est perméable, de préférence perforée (Fig. 7).

6. Chambre selon la revendication 5 caractérisée en ce que la perméabilité de la paroi du corps creux (1) s'accroît avec la distance par rapport à la zone enfoncée (5).

7. Chambre selon l'une quelconque des revendications 1 à 6 caractérisée en ce que la paroi intérieure du corps creux (1), qui se situe entre l'excroissance en forme d'entonnoir (3) et la zone enfoncée (5) et latéralement de l'ouverture d'admission (2), forme une cloison (6) de faible hauteur qui sépare lesdites zones.

8. Chambre selon la revendication 1 caractérisée en ce que le volume du corps creux est compris entre environ 55 et 75 cm³.

9. Chambre selon la revendication 3 caractérisée en ce que la contenance de la zone enfoncée en forme d'auge est de l'ordre de 3 cm³, en position d'utilisation de la chambre.

10. Chambre selon les revendications 5 et 6 caractérisée en ce que la zone adjacente à la zone (5) enfoncée, en forme d'auge, comporte de nombreuses perforations ou perçages présentant un diamètre d'environ 0,5 mm.

11. Chambre selon la revendication 5 ou 6 caractérisée en ce que la zone présentant une perméabilité accrue de la paroi comporte de nombreuses perforations et perçages ayant un diamètre d'environ 1,5 mm.

12. Chambre selon l'une quelconque des revendications 1 à 4 caractérisée en ce qu'un bord (10) faisant saillie à l'intérieur du corps creux (1) et s'étendant sur la périphérie de la paroi inférieure est prévu en deça de l'ouverture d'admission (2) ainsi qu'au-delà de l'excroissance (3) en forme d'entonnoir et de la zone enfoncée (5) en forme d'auge (Fig. 9).

13. Chambre selon la revendication 12 caractérisée en ce que le bord (10) dépasse plus dans la zone située au-delà de l'excroissance en forme d'entonnoir (3) que dans le reste de son parcours.

14. Chambre selon la revendication 1 caractérisée en ce que le corps creux (1) présente une dimension qui correspond approximativement à un oeuf de poule moyen.

15. Chambre selon la revendication 1 caractérisée en ce que le bord (7) de l'ouverture d'admission (2), reliable à l'utérus (9) est constitué par un autre matériau que le corps creux lui-même.

Fig.1 Fig.2 Fig.3 Fig.4 Fig.5 Fig.6

Fig. 7

Fig. 8

Fig. 9

Fig.10

Fig.11

0 166 979

Fig. 12

Fig. 13

7